# EUROPEAN PATENT APPLICATION

(11) **EP 3 770 253 A1**
(43) Date of publication of application: **27.01.2021**
(21) Application number: 19771940.4
(22) Date of filing: 18.03.2019
(51) Int. Cl.: C12N 5/16, A61K 35/39, C12N 11/04

(54) **HYDROGEL CAPSULE**

(30) Priority: 19.03.2018 JP 2018051297
(71) Applicant: Kyoto University, Kyoto-shi, Kyoto 606-8501 (JP); Takeda Pharmaceutical Company Limited, Osaka-shi, Osaka 541-0045, (JP)
(72) Inventor: TOYODA, Taro, Kyoto-shi, Kyoto 606-8501 (JP); MOCHIDA, Taisuke, Fujisawa-shi, Kanagawa 251-0012 (JP); TANOUE, Yutaka, Fujisawa-shi, Kanagawa 251-0012 (JP); ITO, Ryo, Fujisawa-shi, Kanagawa 251-0012 (JP); KIMOTO, Koya, Fujisawa-shi, Kanagawa 251-0012 (JP); KOIKE, Yusuke, Fujisawa-shi, Kanagawa 251-0012 (JP); UENO, Hikaru, Fujisawa-shi, Kanagawa 251-0012 (JP)
(74) Representative: Jones, Nicholas Andrew
(86) International application number: PCT/JP2019/012289
(87) International publication number: WO 2019/182157

(57) **Abstract**

An object of the present invention is to provide a method for removing highly proliferative Ki67-positive cells co-present in insulin-secreting cells obtained by differentiation induction. A method for producing an insulin-producing cell population or a pancreatic beta cell population containing less than 3% of Ki67-positive cells, comprising: embedding an endocrine progenitor cell population or a cell population at a later stage of differentiation into a gel containing alginic acid; and differentiating the cell population.

## Description

### Technical field

The present invention relates to a method for removing highly proliferative Ki67-positive cells present in an insulin-producing cell population or a pancreatic beta cell population obtained from pluripotent stem cells by differentiation induction.

### Background Art

Studies have been conducted for applying insulin-secreting cells such as insulin-producing cells and pancreatic beta cells, which are obtained from pluripotent stem cells, such as iPS cells and ES cells by differentiation induction, to treatment of diabetes.

Up to present, various techniques for inducing differentiation of pluripotent stem cells to insulin-secreting cells have been developed and reported. Non Patent Literature 1 discloses that pancreatic progenitor cells derived from human ES cells can be cultured by using an alginic acid-based scaffold, and confirms that expression of insulin PDX1 is stabilized or decreased; expression of glucagon/somatostatin increased; and expression of β-cell associated genes decreased, in all culture conditions tested.

Also, methods for transplanting insulin-secreting cells into a living organism have been studied. It is reported that interference of a recipient's immune system can be avoided by enclosing pancreatic islets within alginate hydrogel and transplanting such an enclosure system into a living organism (Patent Literatures 1, 2).

It has not yet been known that highly proliferative cells are present in an insulin-producing cell population or a pancreatic beta cell population, which is obtained by further inducing differentiation of an endocrine progenitor cell population derived from pluripotent stem cells or a cell population at a later stage of differentiation, and no attempt has been made to remove such highly proliferative cells.

### Citation List

### Patent Literatures

Patent Literature 1: WO2010/032242
Patent Literature 2: WO2011/154941

### Non Patent Literatures

Non Patent Literature 1: J Biomed Mater Res A. 2015 Dec; 103 (12): 3717-26.

### Summary of Invention

### Technical Problem

The present inventors found that, in a cell population of insulin-producing cells or pancreatic beta cells, which are obtained from pluripotent stem cells by differentiation induction, highly proliferative cells (hereinafter referred to as "Ki67-positive cells") characterized by being positive to Ki67 marker are co-present in the insulin-secreting cells (insulin-producing cells and pancreatic beta cells).

When the insulin-secreting cells obtained by differentiation induction are applied to e.g., treatment of diabetes, it is extremely important, from a safety point of view, to strictly control cells except the insulin-secreting cells. Contamination with highly proliferative cells and remaining thereof are undesirable because an adverse effect on a recipient and an effect on long-term engraftment of transplanted insulin-secreting cells may be produced.

Then, an object of the present invention is to provide a method for removing highly proliferative Ki67-positive cells co-present in insulin-secreting cells obtained by differentiation induction.

### Solution to Problem

The present inventors conducted intensive studies in a view to attaining the above object. As a result, they found that an insulin-producing cell population or a pancreatic beta cell population decreased in the content of Ki67-positive cells can be obtained by embedding an endocrine progenitor cell population obtained from pluripotent stem cells by differentiation induction or a cell population at a later stage of differentiation into a gel containing alginic acid, and further differentiating the cell population, thereby decreasing the number of Ki67-positive cells or inhibiting the proliferation of the cells.

The present invention is based on these new findings and includes the following inventions.
[1] A method for producing an insulin-producing cell population or a pancreatic beta cell population containing less than 3% of Ki67-positive cells, comprising the steps of:
   (1) embedding an insulin-producing cell population into a gel containing alginic acid; and (2) differentiating the cell population embedded.
      [1-1] A method for producing an insulin-producing cell population or a pancreatic beta cell population containing less than 3% of Ki67-positive cells, comprising the steps of:
         (1) embedding an endocrine progenitor cell population or a cell population at a later stage of differentiation into a gel containing alginic acid; and (2) differentiating the cell population embedded.
      [1-2] The method according to [1] or [1-1], which is a method for producing an insulin-producing cell population or a pancreatic beta cell population containing less than 1% of Ki67-positive cells.
[2] The method according to [1] or [1-1], wherein the gel in step (1) further contains a nanofiber.
[3] The method according to [1] or [2], wherein the differentiation in step (2) is performed by transplantation to an animal.
[4] A method for decreasing the number of Ki67-positive cells present in an endocrine progenitor cell population or a cell population at a later stage of differentiation or a method for inhibiting the proliferation of the positive cells, comprising
   embedding the cell population into a gel containing alginic acid.
   [4-1] A method for inhibiting the proliferation of Ki67-positive cells present in an endocrine progenitor cell population or a cell population at a later stage of differentiation, comprising
      embedding the cell population into a gel containing alginic acid.
   [4-2] The method according to [4], wherein the absolute number of Ki67-positive cells present in the cell population is decreased.
[5] The method according to any one of [4] to [4-2], wherein the number of the endocrine progenitor cells or the cells at a later stage of differentiation present in the cell population is not decreased.
   [5-1] The method according to any one of [4] to [4-2], wherein the number of the endocrine progenitor cells or the cells at a later stage of differentiation, which are not Ki67-positive cells, present in the cell population is not decreased.
[6] The method according to any one of [4] to [5-1], wherein the gel further contains a nanofiber.
[7] A gel containing alginic acid into which an insulin-producing cell population or a pancreatic beta cell population containing less than 3% of Ki67-positive cells is embedded.
   [7-1] The gel containing alginic acid according to [7], in which the Ki67-positive cells in the cell population are less than 1%.
   [7-2] The gel containing alginic acid according to [7], in which the cell population is a cell population derived from pluripotent stem cells.
   [7-3] The gel containing alginic acid according to [7], for use in a method for treating diabetes.
[8] The gel containing alginic acid according to [7], further containing a nanofiber.
[9] A method for producing an insulin-producing cell population or a pancreatic beta cell population containing less than 3% of Ki67-positive cells, comprising the steps of:
   (0) containing any one of the methods described in the specification (for example, any one of the methods described in paragraphs [0055] to [0086]),
   (1) embedding an insulin-producing cell population into a gel containing alginic acid; and
   (2) differentiating the cell population embedded.
[10] A method for treating diabetes by immobilizing a gel containing alginic acid into which an insulin-producing cell population or a pancreatic beta cell population containing less than 3% of Ki67-positive cells is embedded, within a living body.
[11] A gel containing alginic acid for inhibiting the proliferation of Ki67-positive cells present in an endocrine progenitor cell population or a cell population at a later stage of differentiation.
[12] Use of gel containing alginic acid in producing a medicament containing an insulin-producing cell population or a pancreatic beta cell population containing less than 3% of Ki67-positive cells.
[13] A method for producing an insulin-producing cell population or a pancreatic beta cell population containing less than 3% of Ki67-positive cells, comprising the steps of:
   (1) purifying cells;
   (2) embedding an insulin-producing cell population into a gel containing alginic acid; and
   (3) differentiating the cell population embedded.

The specification incorporates the contents of the specification and/or drawings of Japanese Patent Application No. 2018-051297, based on which the priority of the present application is claimed.

All publications, patents and patent applications cited herein are incorporated in their entirety by reference.

### Advantageous Effects of Invention

According to the present invention, it is possible to provide a method for removing highly proliferative Ki67-positive cells co-present in insulin-secreting cells obtained by induction of differentiation.

### Brief Description of Drawings

[Figure 1] Figure 1 includes photographs showing a Ki67-positive cell proliferation inhibitory effect on day 69 after transplantation of endocrine progenitor cells embedded into a gel containing alginic acid.
[Figure 2] Figure 2 includes graphs showing the results of flow cytometry of cells recovered from a graft excised out 6 months after transplantation. (A) shows the proportion of desired cells, insulin positive/NKX6.1 positive cells before and after transplantation; and (B) shows the proportion of undesired cells Ki67 positive/insulin negative cells before and after transplantation.

### Description of Embodiments

### 1. Terms

Terms used in the specification will be described.

In the specification, "about" refers to a value which may vary up to plus or minus 25%, 20%, 10%, 8%, 6%, 5%, 4%, 3%, 2%, or 1% from the reference value. Preferably, the term "about" or "around" refers to a range from minus or plus 15%, 10%, 5%, or 1% from the reference value.

In the specification, "comprise(s)" or "comprising" means inclusion of the element(s) following the word without limitation thereto. Accordingly, it indicates inclusion of the element(s) following the word, but does not indicate exclusion of any other element.

In the specification, "consist(s) of" or "consisting of" means inclusion of all the element(s) following the phrase and limitation thereto. Accordingly, the phrase "consist(s) of" or "consisting of" indicates that the enumerated element(s) is required or essential and substantially no other elements exist.

In the specification, without "using feeder cells" means basically containing no feeder cells and using no medium preconditioned by culturing feeder cells. Accordingly, the medium does not contain any substance, such as a growth factor or a cytokine, secreted by feeder cells.

"Feeder cells" or "feeder" means cells that are co-cultured with another kind of cells, support the cells, and provide an environment that allows the cells to grow. The feeder cells may be derived from the same species as or a different species from the cells that they support. For example, as a feeder for human cells, human skin fibroblasts or human embryonic-stem cells may be used or a primary culture of murine embryonic fibroblasts or immortalized murine embryonic fibroblasts may be used. The feeder cells can be inactivated by exposure to radiation or treatment with mitomycin C.

In the specification, "adhered " or "adherent" refers to cells attaching to a container, for example, cells attaching to a cell culture dish or a flask made of a sterilized plastic (or coated plastic) in the presence of an appropriate medium. Some cells cannot be maintained or grow in culture without adhering to the cell culture container. In contrast, non-adherent cells can be maintained and proliferate in culture without adhering to the container.

In the specification, "culture" refers to maintaining, proliferating (growing), and/or differentiating cells in in vitro environment. "Culture(s)" or "culturing" means maintaining, proliferating (growing), and/or differentiating cells out of tissue or the living body, for example, in a cell culture dish or flask.

In the specification, "enrich(es)" and "enrichment" refer to increasing the amount of a certain component in a composition such as a composition of cells and "enriched" refers, when used to describe a composition of cells, for example, a cell population, to a cell population increased in the amount of a certain component in comparison with the percentage of such component in the cell population before the enrichment. For example, a composition such as a cell population can be enriched for a target cell type and, accordingly, the percentage of the target cell type is increased in comparison with the percentage of the target cells present in the cell population before the enrichment. A cell population can be enriched for a target cell type by a method of selecting and sorting cells known in the art. A cell population can be enriched by a specific process of sorting or selection described herein. In a certain embodiment of the present invention, a cell population is enriched for a target cell population at least 20%, 30%, 40%, 50%, 60%, 70%, 80%, 85%, 90%, 95%, 97%, 98%, or 99% by a method of enriching the target cell population.

In the specification, "deplete(s)" and "depletion" refer to decreasing the amount of a certain component in a composition such as cells or a composition of cells and "depleted" refers, when used to describe cells or a composition of cells, for example, a cell population, to a cell population decreased in the amount of a certain component in comparison with the percentage of such component in the cell population before the depletion. For example, a composition such as a cell population can be depleted for a target cell type and, accordingly, the percentage of the target cell type is decreased in comparison with the percentage of the target cells present in the cell population before the depletion. A cell population can be depleted for a target cell type by a method of selecting and sorting cells known in the art. A cell population can be depleted by a specific process of sorting or selection described herein. In a certain embodiment of the present invention, a cell population is reduced (depleted) for a target cell population at least 50%, 80%, 85%, 90%, 95%, 97%, 98%, or 99% by a method of depleting a target cell population.

In the specification, "the number of cells is not decreased" means that the number of cells does not significantly decrease by application of the method of the present invention, and that the number of cells does not significantly change before and after application of the method. However, the number of cells may decrease by a cause not due to application of the method of the present invention (for example, natural death of cells that usually occurs during cell culture conventionally known and differentiation step). Accordingly, the case where "the number of cells is not decreased" includes the case where a decrease ratio of the cells after to before the method of the present invention was applied is 30% or less, 20% or less, 10% or less, or 5% or less.

In the specification, "marker" means a cell antigen that is specifically expressed by a predetermined cell type or a gene thereof, such as "marker protein" and "marker gene". Preferably, a marker is a cell surface marker and this allows concentration, isolation, and/or detection of living cells. A marker can be a positive selection marker or a negative selection marker.

The detection of a marker protein can be conducted by an immunological assay, for example, ELISA, immunostaining, or flow cytometry using an antibody specific for the marker protein. The detection of a marker gene can be conducted by a method of amplifying and/or detecting nucleic acid known in the art, for example, RT-PCR, microarray, biochip, or the like. As used herein, a marker protein being "positive" means that the marker protein is detected to be positive by flow cytometry; whereas, a marker protein being "negative" means that the level of the marker protein is a detection limit or less by flow cytometry. A marker gene being "positive" means that the marker gene is detected by RT-PCR; whereas, a marker gene being "negative" means that the level of the marker gene is detection limit or less by RT-PCR.

In the specification, "expression" is defined as transcription and/or translation of a certain nucleotide sequence driven by an intracellular promoter.

In the specification, the "factor having a CDK8/19 inhibitory activity" means any one of the substances having a CDK8/19 inhibitory activity. CDK8, in contrast to other proteins belonging to the same CDK family, is not required for cell proliferation. For the reason, inhibition of CDK8 does not produce a significant effect under normal conditions. CDK19 is analogous to CDK8 as described above and inhibition of CDK8 usually entails inhibition of CDK19.

"Growth factors" are endogenous proteins that promote differentiation and/or proliferation of particular cells. Examples of "growth factors" include epidermal growth factor (EGF), acid fibroblast growth factor (aFGF), basic fibroblast growth factor (bFGF), hepatocyte growth factor (HGF), insulin-like growth factor 1 (IGF-1), insulin-like growth factor 2 (IGF-2), keratinocyte growth factor (KGF), nerve growth factor (NGF), platelet-derived growth factor (PDGF), transformation growth factor beta (TGF-β), vascular endothelial growth factor (VEGF), transferrin, various interleukins (for example, IL-1 to IL-18), various colony-stimulating factors (for example, granulocyte/macrophage colony-stimulating factor (GM-CSF)), various interferons (for example, IFN-γ, and the like), and other cytokines having effects on stem cells, for example, stem cell factor (SCF), and erythropoietin (Epo).

In the specification, "ROCK inhibitors" means substances that inhibit Rho kinase (ROCK: Rho-associated, coiled-coil containing protein kinase) and may be substances that inhibit either of ROCK I and ROCK II. The ROCK inhibitors are not particularly limited as long as they have the aforementioned function and examples include N-(4-pyridinyl)-4β-[(R)-1-aminoethyl]cyclohexane-1α-carboxamide (that may be herein also referred to as Y-27632), Fasudil (HA1077), (2S)-2-methyl-1-[(4-methyl-5-isoquinolinyl]sulfonyl]hexahydro-1H-1,4-diazepine (H-1152), 4β-[(1R)-1-aminoethyl]-N-(4-pyridyl)benzene-1zenecarboxamide (Wf-536), N-(1H-pyrrolo[2,3-b]pyridin-4-yl)-4PER(R)-1-aminoethyl]cyclohexane-1α-carboxamide (Y-30141), N-(3-{[2-(4-amino-1,2,5-oxadiazol-3-yl)-1-ethyl-1H-imidazo[4,5-c]pyridin-6-yl]oxy}phenyl)-4-{[2-(4-morpholinyl)ethyl]-oxy}benzamide (GSK269962A), N-(6-fluoro-1H-indazol-5-yl)-6-methyl-2-oxo-4-[4-(trifluoromethyl)phenyl]-3,4-dihydro-1H-pyridine-5-carboxamide (GSK429286A). The ROCK inhibitors are not limited to these and antisense oligonucleotides and siRNA to ROCK mRNA, antibodies that bind to ROCK, and dominant negative ROCK mutants can also be used as ROCK inhibitors, and commercially available, or synthesized according to a known method.

In the specification, "GSK3β inhibitors" are substances having the inhibitory activity for GSK3β (glycogen synthase kinase 3β). GSK3 (glycogen synthase kinase 3) is a serine/threonine protein kinase and involved in many signaling pathways associated with the production of glycogen, apoptosis, maintenance of stem cells, etc. GSK3 has the 2 isoforms α and β. "GSK3β inhibitors" used in the present invention are not particularly limited as long as they have the GSK3β-inhibiting activity and they may be substances having both the GSK3α-inhibiting activity and the GSK3β-inhibiting activity.

Examples of GSK3β inhibitors include CHIR98014 (2-[[2-[(5-nitro-6-aminopyridin-2-yl)amino]ethyl]amino]-4-(2,4-dichlorophenyl)-5-(1H-imidazol-1-yl)pyrimidine), CHIR99021 (6-[[2-[[4-(2,4-dichlorophenyl)-5-(4-methyl-1H-imidazol-2-yl)-2-pyrimidinyl]amino]ethyl]amino]nicotinonitrile), TDZD-8 (4-benzyl-2-methyl-1,2,4-thiadiazolidine-3,5-dione), SB216763 (3-(2,4-dichlorophenyl)-4-(1-methyl-1H-indol-3-yl)-1H-pyrrole-2,5-dione), TWS-119 (3-[6-(3-aminophenyl)-7H-pyrrolo[2,3-d]pyrimidin-4-yloxy]phenol), kenpaullone, 1-azakenpaullone, SB216763 (3-(2,4-dichlorophenyl)-4-(1-methyl-1H-indol-3-yl)-1H-pyrrole-2,5-dione), SB415286 (3-[(3-chloro-4-hydroxyphenyl)amino]-4-(2-nitrophenyl)-1H-pyrrole-2,5-dione), and AR-AO144-18, CT99021, CT20026, BIO, BIO-acetoxime, pyridocarbazole-ruthenium cyclopentadienyl complex, OTDZT, alpha-4-dibromoacetophenone, lithium, and the like. GSK3β is not limited to these and antisense oligonucleotides and siRNA to GSK3β mRNA, antibodies that bind to GSK3β, dominant negative GSK3β mutants, and the like can also be used as GSK3β inhibitors, and commercially available, or synthesized according to a known method.

In the specification, examples of "serum replacement" include Knockout Serum Replacement (KSR: Invitrogen), StemSure Serum Replacement (Wako), B-27 supplement, N2-supplement, albumin (for example, lipid rich albumin), insulin, transferrin, fatty acids, collagen precursors, trace elements (for example, zinc, selenium (for example, sodium selenite)), 2-mercaptoethanol, 3'-thiolglycerol, or mixtures thereof (for example, ITS-G). Preferred serum replacements are B-27 supplement, KSR, StemSure Serum Replacement, ITS-G. The concentration of serum replacement in a medium when added into a medium is 0.01-10% by weight, and preferably 0.1-2% by weight. In the present invention, "serum replacement" is preferably used instead of serum.

### 2. Insulin-producing cell population or a pancreatic beta cell population in which the proliferation of Ki67-positive cells is inhibited.

The present invention relates to an insulin-producing cell population or a pancreatic beta cell population in which the proliferation of Ki67-positive cells is inhibited. These cell populations can be obtained by embedding an endocrine progenitor cell population or a cell population at a later stage of differentiation into a gel containing alginic acid and differentiating the cell population into a desired cell population.

In the specification, the "Ki67-positive cells" refer to cells which are characterized by expression of Ki67 as a marker during the differentiation process from pluripotent stem cells into pancreatic β cells and coexist at least within an endocrine progenitor cell population or a cell population at a later stage of differentiation. "Ki67" is known as a cell cycle-associated nuclear protein. Since expression thereof is confirmed in G1 phase, S phase, G2 phase and M phase in cells during proliferation and not confirmed in a resting phase of cell proliferation, G0 phase, "Ki67" is known as a marker of cell proliferation and a cell cycle.

During a process of differentiation from pluripotent stem cells into pancreatic β cells, cells varying in characteristic depending on the differentiation stage are known to emerge (WO2009/012428, WO2016/021734). For example, the cells can be roughly classified into pluripotent stem cells, intraembryonic endoderm cells, archenteric canal cells, posterior foregut cells, pancreatic progenitor cells, endocrine progenitor cells, insulin-producing cells and pancreatic beta cells in ascending order of differentiation.

In the specification, "pluripotency" means the ability to differentiate into tissues and cells having various different shapes and functions and to differentiate into cells of any lineage of the 3 germ layers. "Pluripotency" is different from "totipotency", which is the ability to differentiate into any tissue of the living body, including the placenta, in that pluripotent cells cannot differentiate into the placenta and therefore, do not have the ability to form an individual.

In the specification, "multipotency" means the ability to differentiate into plural and limited numbers of linages of cells. For example, mesenchymal stem cells, hematopoietic stem cells, neural stem cells are multipotent, but not pluripotent.

In the specification, "Pluripotent stem cells" refers to embryonic-stem cells (ES cells) and cells potentially having a pluripotency similar to that of ES cells, that is, the ability to differentiate into various tissues (all of the endodermal, mesodermal, and ectodermal tissues) in the living body. Examples of cells having a pluripotency similar to that of ES cells include "induced pluripotent stem cells" (that may be herein also referred to as "iPS cells"). In the present invention, "Pluripotent stem cells" is preferably human pluripotent stem cells.

Available "ES cells" include murine ES cells, such as various murine ES cell lines established by inGenious targeting laboratory, RIKEN (Institute of Physical and Chemical Research), and the like, and human ES cells, such as various human ES cell lines established by NIH, RIKEN, Kyoto University, Cellartis, and the like. For example, available human ES cell lines include NIH cell lines CHB-1 to CHB-12, RUES1, RUES2, HUES1 to HUES28, and the like; WisCell Research cell lines H1 and H9; and RIKEN cell lines KhES-1, KhES-2, KhES-3, KhES-4, KhES-5, SSES1, SSES2, SSES3, and the like.

"Induced pluripotent stem cells" refers to cells that are obtained by reprograming mammalian somatic cells or undifferentiated stem cells by introducing particular factors (nuclear reprogramming factors). At present, there are various "induced pluripotent stem cells" and iPS cells established by Yamanaka, et al. by introducing the 4 factors Oct3/4, Sox2, Klf4, c-Myc into murine fibroblasts (Takahashi K, Yamanaka S., Cell, (2006) 126: 663-676); iPS cells derived from human cells, established by introducing similar 4 factors into human fibroblasts (Takahashi K, Yamanaka S., et al. Cell, (2007) 131: 861-872.); Nanog-iPS cells established by sorting cells using expression of Nanog as an indicator after introduction of the 4 factors (Okita, K., Ichisaka, T., and Yamanaka, S. (2007). Nature 448, 313-317.); iPS cells produced by a method not using c-Myc (Nakagawa M, Yamanaka S., et al. Nature Biotechnology, (2008) 26, 101-106); and iPS cells established by introducing 6 factors in a virus-free way (Okita K et al. Nat. Methods 2011 May; 8(5): 409-12, Okita K et al. Stem Cells. 31 (3) 458-66) may be also used. Also, induced pluripotent stem cells established by introducing the 4 factors OCT3/4, SOX2, NANOG, and LIN28 by Thomson et al. (Yu J., Thomson JA. et al., Science (2007) 318: 1917-1920.); induced pluripotent stem cells produced by Daley et al. (Park IH, Daley GQ.et al., Nature (2007) 451: 141-146); induced pluripotent stem cells produced by Sakurada et al. (Japanese Unexamined Patent Application Publication No. 2008-307007) and the like may be used.

In addition, any of known induced pluripotent stem cells known in the art described in all published articles (for example, Shi Y., Ding S., et al., Cell Stem Cell, (2008) Vol 3, Issue 5, 568-574; Kim JB., Scholer HR., et al., Nature, (2008) 454, 646-650; Huangfu D., Melton, DA., et al., Nature Biotechnology, (2008) 26, No. 7, 795-797) or patents (for example, Japanese Unexamined Patent Application Publication No. 2008-307007, Japanese Unexamined Patent Application Publication No. 2008-283972, US2008-2336610, US2009-047263, WO2007-069666, WO2008-118220, WO2008-124133, WO2008-151058, WO2009-006930, WO2009-006997, WO2009-007852) may be used.

Available induced pluripotent cell lines include various iPS cell lines established by NIH, RIKEN (Institute of Physical and Chemical Research), Kyoto University and the like. For example, such human iPS cell lines include the RIKEN cell lines HiPS-RIKEN-1A, HiPS-RIKEN-2A, HiPS-RIKEN-12A, and Nips-B2 and the Kyoto University cell lines Ff-WJ-18, Ff-I01s01, Ff-I01s02, Ff-I01s04, Ff-I01s06, Ff-I14s03, Ff-I14s04, QHJI01s01, QHJI01s04, QHJI14s03, QHJI14s04, 253G1, 201B7, 409B2, 454E2, 606A1, 610B1, 648A1, the CDI cell lines Mycell iPS Cells (21525.102.10A), Mycell iPS Cells (21526.101.10A) and the like.

In the specification, the "endocrine progenitor cell population" refers to a cell population characterized by endocrine progenitor cells.

The endocrine progenitor cells refer to cells characterized by expression of at least one of the markers, Chromogranin A, NeuroD and NGN-3, and non-expression of pancreas associated hormon-related marker (for example, insulin). In the endocrine progenitor cells, markers such as PAX-4, NKX2-2, Islet-1, PDX-1 and PTF-1α may be expressed.

The endocrine progenitor cell population is a cell population containing endocrine progenitor cells at a proportion of 30% or more, preferably 50% or more, and more preferably 70% or more.
In the endocrine progenitor cell population, other than the endocrine progenitor cells, cells (for example, pancreatic progenitor cells, insulin-producing cells, Ki67-positive cells) may be included.

The proportion of predetermined cells in a cell population can be obtained based on a known method for determining the number of cells, such as flow cytometry.

The "cell population at a later stage of differentiation" refers to a cell population characterized by the cells further differentiated from endocrine progenitor cells towards pancreatic β cells. Examples of the cells further differentiated from endocrine progenitor cells towards pancreatic β cells, include insulin-producing cells.

The "insulin-producing cells" refer to cells characterized by expression of insulin as a marker. The "insulin-producing cells" may express a marker of NKX6.1, and preferably express both markers, i.e., insulin and NKX6.1.

The "cell population at a later stage of differentiation" or the "insulin-producing cell population" is a cell population containing insulin-producing cells at a proportion of 5% or more, preferably 15% or more, and more preferably 30% or more. The cell population may include, other than insulin-producing cells, cells (for example, endocrine progenitor cells; other pancreatic hormone producing cells expressing at least one of the markers glucagon, somatostatin and pancreatic polypeptide; and Ki67-positive cells).

In the specification, the "pancreatic β cells" refer to more mature cells than "insulin-producing cells"; more specifically, refer to cells characterized by expressing at least one of maturation markers for pancreatic β cells, such as MAFA, UCN3 and IAPP or by an increased insulin secretion reaction by stimulation with glucose.

The "pancreatic β cell population" is a cell population containing pancreatic β cells, which can be obtained by differentiation and maturation of an endocrine progenitor cell population or a cell population at a later stage of differentiation in a living organism. The cell population may contain, other than pancreatic β cells, cells (for example, insulin-producing cells and Ki67-positive cells).

In the present invention, the proportion of Ki67-positive cells in the cells (starting material cells) to be embedded into a gel containing alginic acid, although it is not particularly limited, may be 80% to 0.1%, 70% to 1%, 60% to 2%, 50% to 3%, 40% to 4% or 30% to 5%.

In the present invention, the expression level of MAFA gene or its protein in the cells (starting material cells) to be embedded into a gel containing alginic acid, although it is not particularly limited, may be about 20% or less, about 15% or less, about 10% or less, about 5% or less, or about 1% or less of the expression level of MAFA gene or its protein in pancreatic islets.

In the present invention, the proportion of glucagon-positive and insulin-negative cells in the cells (starting material cells) to be embedded into a gel containing alginic acid, although it is not particularly limited, may be about 2.5% or less, about 2% or less, about 1% or less, or about 0.5% or less.

In the present invention, the proportion of Chromogranin A-positive cells in the cells (starting material cells) to be embedded into a gel containing alginic acid, although it is not particularly limited, may be about 50% or more (for example, about 55% or more), about 60% or more, about 70% or more, about 80% or more, or about 90% or more.

In the present invention, the proportion of alkaline phosphatase-positive cells in the cells (starting material cells) to be embedded into a gel containing alginic acid, although it is not particularly limited, may be 1% or less, 0.5% or less, 0.01% or less, 0.008% or less, 0.005% or less, or 0.001% or less.

In the present invention, the proportion of Ki67-positive cells in the cells (starting material cells) to be embedded into a gel containing alginic acid may be about 50% or less, or about 30% or less; the expression level of MAFA gene or its protein may be about 20% or less, or about 10% or less of the expression level of MAFA gene or its protein in pancreatic islets; the proportion of glucagon-positive and insulin-negative cells may be about 2.5% or less, or about 1% or less; the proportion of Chromogranin A-positive cells may be about 50% or more, and the proportion of alkaline phosphatase positive cells may be 1% or less.

The endocrine progenitor cell population or cell population at a later stage of differentiation can be obtained by use of a known differentiation induction technique for pluripotent stem cells into pancreatic β cells. More specifically, individual desired cell populations can be obtained by the following differentiation induction steps:
step 1) inducing a differentiation of pluripotent stem cells into intraembryonic endoderm cells;
step 2) inducing a differentiation of the intraembryonic endoderm cells into archenteric canal cells;
step 3) inducing a differentiation of the archenteric canal cells into posterior foregut cells;
step 4) inducing a differentiation of the posterior foregut cells into pancreatic progenitor cells;
step 5) inducing a differentiation of the pancreatic progenitor cells into endocrine progenitor cells;
step 6) inducing a differentiation of the endocrine progenitor cells into insulin-producing cells.

Now, each of the steps will be described below; however, differentiation induction into each of the types of cells is not limited to these techniques described below.

### Step 1) Differentiation into intraembryonic endoderm cells

Pluripotent stem cells are first differentiated into intraembryonic endoderm cells. Methods for inducing pluripotent stem cells into intraembryonic endoderm are already known and any one of the methods may be used. Preferably, pluripotent stem cells are cultured in a medium containing activin A, more preferably activin A, a ROCK inhibitor, and a GSK3 β inhibitor, to differentiate into intraembryonic endoderm cells. The number of cells at the initiation time of culture, although it is not particularly limited, is 22000 to 150000 cells/cm², preferably 22000 to 100000 cells/cm² and more preferably 22000 to 80000 cells/cm². The culture period is 1 to 4 days, preferably 1 to 3 days, and particularly preferably 3 days.

The culture temperature, although it is not particularly limited, is 30 to 40°C (for example, 37°C). The concentration of carbon dioxide in a culture vessel is, for example, about 5%.

Examples of the culture medium that can be used in this step include basic mediums to be used for culturing of mammalian cells, such as RPMI 1640 medium, MEM medium, iMEM medium, DMEM/F12 medium and improved MEM Zinc Option medium.

The concentration of activin A in a medium is usually 30 to 200 ng/mL, preferably 50 to 150 ng/mL, more preferably 70 to 120 ng/mL, and particularly preferably about 100 ng/mL.

In another embodiment, the concentration of activin A in a medium is about 0.1 to 100 ng/mL, preferably about 1 to 50 ng/mL and more preferably about 3 to 10 ng/mL.

The concentration of the GSK3 β inhibitor in a medium is appropriately determined depending on the type of GSK3 β inhibitor to be used. For example, if CHIR99021 is used as the GSK3 β inhibitor, the concentration thereof is usually 1 to 10 µM, preferably 2 to 5 µM, more preferably 2 to 4 µM and particularly preferably about 3 µM.

The concentration of the ROCK inhibitor in a medium is appropriately determined depending on the type of ROCK inhibitor to be used. For example, if Y27632 is used as the ROCK inhibitor, the concentration thereof is usually 5 to 20 µM, preferably 5 to 15 µM and particularly preferably about 10 µM.

More specifically, pluripotent stem cells are cultured in a medium containing activin A, a ROCK inhibitor and a GSK3 β inhibitor for a day and thereafter cultured in a medium containing activin A alone for further two days while exchanging the medium every day.

### Step 2) Differentiation into archenteric canal cells

The intraembryonic endoderm cells obtained in step 1) are further cultured in a medium containing a growth factor to induce differentiation into archenteric canal cells. The culture period is 2 to 8 days and preferably about 4 days.

The culture temperature, although it is not particularly limited, is 30 to 40°C (for example, 37°C). The carbon dioxide concentration in a culture vessel is, for example, about 5%.

As the culture medium, a basic medium for culturing mammalian cells can be used similarly to the case of step 1). To the medium, substances other than a growth factor, such as a serum substitute, a vitamin and an antibiotic substance, may be appropriately added.

As the growth factor, EGF, KGF and FGF10 are preferable, EGF and/or KGF are more preferable, and KGF is further preferable.

The concentration of the growth factor in a medium can be appropriately determined depending on the type of growth factor to be used. The concentration thereof is usually about 0.1 nM to 1000 µM, and preferably about 0.1 nM to 100 µM. In the case of EGF, its concentration is about 5 to 2000 ng/mL (more specifically, about 0.8 to 320 nM), preferably about 5 to 1000 ng/mL (more specifically, about 0.8 to 160 nM), and more preferably about 10 to 1000 ng/mL (more specifically, about 1.6 to 160 nM). In the case of FGF10, its concentration is about 5 to 2000 ng/mL (more specifically, about 0.3 to 116 nM), preferably about 10 to 1000 ng/mL (more specifically, about 0.6 to 58 nM), and more preferably about 10 to 1000 ng/mL (more specifically, about 0.6 to 58 nM). For example, if KGF is used as a growth factor, the concentration thereof is usually 5 to 150 ng/mL, preferably 30 to 100 ng/mL, and particularly preferably about 50 ng/mL.

### Step 3) Differentiation into posterior foregut cells

The archenteric canal cells obtained in step 2) are further cultured in a medium containing, e.g., a growth factor, cyclopamine and noggin, to induce differentiation into posterior foregut cells. The culture period is 1 to 5 days, preferably about 2 days.

The culture temperature, although it is not particularly limited, is 30 to 40°C (for example, 37°C). The concentration of carbon dioxide in a culture vessel is, for example, about 5%.

As the culture medium, a basic medium for culturing mammalian cells can be used similarly to the case of step 1). To the medium, substances other than a growth factor, such as a serum substitute, a vitamin and an antibiotic substance, may be appropriately added.

As the growth factor, EGF, KGF and FGF10 are preferable, EGF and/or KGF are more preferable, and KGF is further preferable.

The concentration of the growth factor in a medium can be appropriately determined depending on the type of growth factor to be used. The concentration thereof is usually about 0.1 nM to 1000 µM and preferably about 0.1 nM to 100 µM. In the case of EGF, its concentration is about 5 to 2000 ng/mL (more specifically, about 0.8 to 320 nM), preferably about 5 to 1000 ng/mL (more specifically, about 0.8 to 160 nM) and more preferably about 10 to 1000 ng/mL (more specifically, about 1.6 to 160 nM). In the case of FGF10, its concentration is about 5 to 2000 ng/mL (more specifically, about 0.3 to 116 nM), preferably about 10 to 1000 ng/mL (more specifically, about 0.6 to 58 nM) and more preferably about 10 to 1000 ng/mL (more specifically, about 0.6 to 58 nM). For example if KGF is used as a growth factor, the concentration thereof is usually 5 to 150 ng/mL, preferably 30 to 100 ng/mL and particularly preferably about 50 ng/mL.

The concentration of cyclopamine in a medium, although it is not particularly limited, is usually 0.5 to 1.5 µM, preferably 0.3 to 1.0 µM and particularly preferably about 0.5 µM.

The concentration of noggin in a medium, although it is not particularly limited, is usually 10 to 200 ng/mL, preferably 50 to 150 ng/mL and particularly preferably about 100 ng/mL.

### Step 4) Differentiation into pancreatic progenitor cells

The posterior foregut cells obtained in step 3) are further cultured in a medium containing, e.g., a factor having a CDK8/19 inhibitory activity, and preferably in a medium containing a factor having a CDK8/19 inhibitory activity and a growth factor to induce differentiation into pancreatic progenitor cells. The culture period is 2 to 10 days and preferably about 5 days.

In accordance with the publication (Toyoda et al., Stem cell Research (2015) 14, 185-197), the posterior foregut cells obtained in step 3) are treated and dispersed by pipetting with 0.25% trypsin-EDTA and the dispersion is subjected to centrifugal separation to obtain cell suspension and then the suspension is reseeded to a fresh medium of step 4.

As the culture medium, a basic medium for culturing mammalian cells can be used similarly to the case of step 1). To the medium, substances other than a growth factor, a serum substitute, a vitamin and an antibiotic substance may be appropriately added.

As the factor having a CDK8/19 inhibitory activity, various compounds as mentioned above or salts thereof can be used. The addition amount of the compound or a salt thereof is appropriately determined depending on the type thereof. The addition amount is usually about 0.00001 µM-5 µM and preferably 0.00001 µM-1 µM. The concentration of the factor having a CDK8/19 inhibitory activity in a medium is preferably a concentration at which 50% or more activity of CDK8/19 is to be inhibited.

As the growth factor, EGF, KGF and FGF10 are preferable, KGF and/or EGF are more preferable, and KGF and EGF are further preferable.

The concentration of the growth factor in a medium can be appropriately determined depending on the type of growth factor to be used. The concentration is usually about 0.1 nM to 1000 µM and preferably about 0.1 nM to 100 µM. In the case of EGF, its concentration is about 5 to 2000 ng/mL (more specifically, about 0.8 to 320 nM), preferably about 5 to 1000 ng/mL (more specifically, about 0.8 to 160 nM) and more preferably about 10 to 1000 ng/mL (more specifically, about 1.6 to 160 nM). In the case of FGF10, its concentration is about 5 to 2000 ng/mL (more specifically, about 0.3 to 116 nM), preferably about 10 to 1000 ng/mL (more specifically, about 0.6 to 58 nM) and more preferably about 10 to 1000 ng/mL (more specifically, about 0.6 to 58 nM). For example if KGF and EGF are used as a growth factor, the concentration of KGF is usually 5 to 150 ng/mL, preferably 30 to 100 ng/mL and particularly preferably about 50 ng/mL; whereas the concentration of EGF is usually 10 to 200 ng/mL, preferably 50 to 150 ng/mL and particularly preferably about 100 ng/mL.

The culture in step 4) may be carried out in the presence of a ROCK inhibitor on the first day and thereafter in a medium without the ROCK inhibitor.

In any one of the steps, other than the aforementioned components, a serum substitute (for example, B-27 supplement, ITS-G) may be added to a medium. If necessary, an amino acid, L-glutamine, GlutaMAX (product name), a nonessential amino acid, a vitamin, an antibiotic substance (for example, Antibiotic-Antimycotic (herein, sometimes referred to as AA), penicillin, streptomycin, or a mixture of these), an antibacterial agent (for example, amphotericin B), an antioxidant, pyruvic acid, a buffer and/or an inorganic salt may be added. In the case of adding an antibiotic substance to a medium, the concentration thereof in the medium is usually 0.01 to 20 wt% and preferably 0.1 to 10 wt%.

Cells are cultured by an adhesion culture without using feeder cells. In culturing, a culture vessel including a dish, a flask, a micro plate and a cell culture sheet such as OptiCell (product name) (company: Nunc) is used. The surface of the culture vessel is preferably treated for improving adhesiveness (hydrophilicity) to cells or coated with a cell adhesion substrate such as collagen, gelatin, poly-L-lysine, poly-D-lysine, laminin, fibronectin, Matrigel (e.g., BD Matrigel (Becton, Dickinson and Company)) and vitronectin. As the culture vessel, a culture vessel coated preferably with, e.g., Type I-collagen, Matrigel, fibronectin, vitronectin or poly-D-lysine is preferable and a culture vessel coated with Matrigel or poly-D-lysine is more preferable.

The culture temperature, although it is not particularly limited, is 30 to 40°C (for example, 37°C). The concentration of carbon dioxide in a culture vessel is, for example, about 5%.

The pancreatic progenitor cells obtained in step 4) can be further purified by use of a known surface marker such as glycoprotein 2 (GP2). The purification can be made by use of a means known per se, for example, beads having an anti-GP2 antibody immobilized thereon.

### Step 5) Differentiation into endocrine progenitor cells

The pancreatic progenitor cell obtained in step 4) are further cultured in a medium containing a growth factor to induce differentiation into endocrine progenitor cells. The culture period is 2 to 3 days and preferably about 2 days.

As the culture medium, a basic medium for culturing mammalian cells can be used similarly to the case of step 1). To the medium, SANT1, retinoic acid, ALK5 inhibitor II, T3 and/or LDN are added in accordance with the publication (Nature Biotechnology 2014; 32: 1121-1133) and further a Wnt inhibitor, a ROCK inhibitor, FGF (preferably FGF2), a serum substitute, a vitamin and/or an antibiotic substance may be added.

Cells are cultured by a non-adhesion culture without using feeder cells. Culture is carried out by using a dish, a flask, a micro plate and perforated plate (company: Nunc) or a bioreactor. The surface of the culture vessel is preferably treated for decreasing adhesiveness to cells.

The culture temperature, although it is not particularly limited, is 30 to 40°C (for example, 37°C). The concentration of carbon dioxide in a culture vessel is, for example, about 5%.

### Step 6) Differentiation into insulin-producing cells

The endocrine progenitor cells obtained in step 5) are further cultured in a medium containing a growth factor to induce differentiation into insulin-producing cells. The culture period is 7 to 15 days and preferably about 7 to 10 days.

As the growth factor, a factor described in the paper (Nature Biotechnology 2014; 32: 1121-1133) is used. In addition, a γ-secretase inhibitor, a Wnt inhibitor, a ROCK inhibitor and/or FGF preferably FGF2 may be added.

As the culture medium, a basic medium for culturing mammalian cells can be used similarly to the case of step 1). To the culture medium, an ALK5 inhibitor II, T3, LDN and/or a γ-secretase inhibitor XX are added in accordance with the publication (Nature Biotechnology 2014;32: 1121-1133), and further, a Wnt inhibitor, a ROCK inhibitor, FGF (preferably FGF2), a serum substitute, a vitamin and/or an antibiotic substance may be appropriately added.

The cells are cultured without using feeder cells, that is, by a non-adhesion culture. In culturing, a culture vessel including a dish, a flask, a micro plate and a perforated plate (company: Nunc) or a bioreactor is used. The surface of the culture vessel is preferably treated for decreasing adhesiveness to cells.

The culture temperature, although it is not particularly limited, is 30 to 40°C (for example, 37°C). The concentration of carbon dioxide in a culture vessel is, for example, about 5%.

In the present invention, the "gel containing alginic acid" can be prepared in accordance with a known method (WO2010/032242, WO2011/154941) and can be obtained by gelatinizing a solution of an alginate, an alginic acid ester or alginic acid modified compound by adding a cross-linking agent.

The alginate is not limited as long as it is a water soluble salt, for example, a metal salt and/or an ammonium salt can be used; for example, sodium alginate, calcium alginate and/or ammonium alginate can be suitably used.

The alginic acid ester (also referred to as propylene glycol alginate) is a derivative obtained by esterifying the carboxyl group of alginic acid with propylene glycol. The alginic acid modified compound is, for example, an RGD modified alginic acid, which is alginic acid modified with a cell adhesion activity sequence (arginine-glycine-aspartic acid).

The proportion of mannuronic acid to guluronic acid contained in an alginate (M/G ratio) (the ratio herein will be sometimes referred to as "grade") is not specified. Generally, if M > G, gel rich in flexibility can be formed; whereas, if M < G, rigid gel can be formed. In the present invention, gel containing guluronic acid at a proportion of 10 to 90%, 20 to 80%, 30 to 70% or 40 to 60% can be used.

An alginate, an alginic acid ester or an alginic acid modified compound can be added in a solvent in an amount of 0.05 to 10 wt%, preferably, 0.1 to 5 wt%, and more preferably 0.5 to 3 wt%. Any solvent can be used as long as it can dissolve an alginate, an alginic acid ester or an alginic acid modified compound; e.g., water and/or saline, can be used.

The cross-linking agent is not particularly limited as long as it can gelatinize a solution of an alginate, an alginic acid ester or an alginic acid modified compound; e.g., a multivalent metal cation can be used. As the multivalent metal cation, a divalent metal cation is preferable and a calcium ion, a strontium ion or a barium ion is more preferable. The cross-linking agent may be used in the form of a salt. In the present invention, at least one selected from calcium chloride, strontium chloride and barium chloride can be used as the cross-linking agent. For crosslinking of Hystem, polyethylene glycol diacrylate may be used.

Gel containing alginic acid may contain a nanofiber. The nanofiber is a natural or synthetic fiber having a diameter within the nanometer range. As the natural nanofiber, a nanofiber containing a single or a plurality of polysaccharides such as collagen, cellulose, silk fibroin, keratin, gelatin and chitosan is mentioned. Examples of the synthetic nanofiber include polylactic acid (PLA), polycaprolactone (PCL), polyurethane (PU), poly(lactide-co-glycolide) (PLGA), poly(3-hydroxybutyrate-co-hydroxyvalerate) (PHBV), and poly(ethylene-co-vinylacetate) (PEVA). The nanofiber can be contained in gel containing alginic acid in an amount of less than 1 wt%, for example, 0.9 wt%, 0.8 wt%, 0.7 wt%, 0.6 wt%, or 0.5 wt% or less. The lower limit of the nanofiber to be added to gel containing alginic acid, although it is not particularly limited, can be 0.05 wt% or more and preferably 0.1 wt% or more.

In the present invention, "embedding" refers to dispersedly putting an endocrine progenitor cell population or a cell population at a later stage of differentiation into a gel containing alginic acid.

A cell population can be embedded into a gel containing alginic acid by mixing the cell population in a solution of an alginate, an alginic acid ester or an alginic acid modified compound and gelatinizing the solution.

A cell population can be added in a solution of an alginate, an alginic acid ester or an alginic acid modified compound in an amount selected from 1 × 10⁴ cells to 1 × 10⁹ cells/mL and preferably 1 × 10⁷ cells to 1 × 10⁸ cells/mL.

A solution of an alginate, an alginic acid ester or an alginic acid modified compound containing a cell population can be gelatinized by adding a cross-linking agent to the solution. The amount of the cross-linking agent to be added can be selected from 0.1 to 5 wt% to the solution, for example, 0.1 to 1 wt%. The gelatinization can be carried out in a container having a predetermined structure and/or shape for use in cell culture and cell transplantation or a mold designed so as to obtain gel fitting the shape of the container.

Alternatively, gelatinization may be carried out by forming gel capsules containing alginic acid in accordance with a known method (WO2010/010902). More specifically, gelatinization may be carried out by adding a solution of an alginate, an alginic acid ester or an alginic acid modified compound containing a cell population dropwise to a solution of a cross-linking agent. The size of droplets can be controlled depending on the shape of a dripping nozzle or the falling-drop method, with the result that the size of gel capsules containing alginic acid can be regulated. Although the falling-drop method is not particularly limited, e.g., an air spray method, an airless spray method and/or an electrostatic spray method can be used. The size of gel capsules containing alginic acid, although it is not particularly limited, can be 5 mm or less, 1 mm or less, or 500 µm or less in diameter. The cross-linking agent may be contained in a crosslinking agent solution in an amount selected from 0.1 to 10 wt%, for example, 0.1 to 5 wt%. The gel capsules containing alginic acid may be covered with membrane having pores of 0.1 µm to 50 µm.

The cell population embedded into a gel containing alginic acid can be subjected to the step of differentiating into a desired cell population. The differentiation step may be carried out by subjecting the cell population to the aforementioned culture method or transplanting the cell population into a living animal. Particularly, a differentiation step into pancreatic β-cell population can be carried out by transplanting an endocrine progenitor cell population or a cell population at a later stage of differentiation embedded into a gel containing alginic acid into a living animal.

The "animal" is preferably a mammal such as a human, a non-human primate, a pig, a cow, a horse, a sheep, a goat, a llama, a dog, a cat, a rabbit, a mouse and a guinea pig; and preferably a human.

Transplantation is preferably carried out into a region of a living body, in which a cell population embedded into a gel containing alginic acid can be immobilized at a predetermined position, for example, the subcutaneous, intraperitoneal, peritoneal epithelium, great omentum, fat tissue and muscle tissue of an animal, and the subcapsule of organs such as pancreas and kidney. A cell population embedded into a gel containing alginic acid can be enclosed in a device such as a capsule, a bag and a chamber and transplanted into a site within a living body. Although the number of cells to be transplanted varies depending on, e.g., the differentiation stage of the cells to be transplanted, the severity of a disease, the age, body weight of the target to be transplanted and size of transplantation site thereof, and not particularly limited; the number of cells can be, for example, about 10 × 10⁴ to 10 × 10¹¹ cells. The cell population transplanted are induced differentiation in the environment of the living body to obtain a desired cell population, preferably a pancreatic β-cell population, which may be recovered later or allowed to remain in the living body as it is.

By embedding an endocrine progenitor cell population or a cell population at a later stage of differentiation into a gel containing alginic acid, and differentiating the cell population, the proliferation of Ki67-positive cells can be inhibited in the cell population.

The method of the invention can be used not for inhibiting the growth of a teratoma but for reducing or inhibiting the residual number of proliferating cells in a pancreatic lineage.

The method of the invention can be used not for inhibiting the proliferation of iPS cells but for reducing or inhibiting the residual number of proliferating cells in a pancreatic lineage.

According to the method of the invention, it is possible to decrease the absolute number of Ki67-positive cells in the cell population obtained after differentiation induction, compared to the case where the cell population is differentiated without being embedded into a gel containing alginic acid. In this manner, Ki67-positive cells in the cell population obtained after differentiation induction can be depleted. More specifically, the proportion of Ki67-positive cells in the cell population obtained after differentiation induction can be decreased, compared to the case where the cells are differentiated without being embedded into a gel containing alginic acid. The ratio can be reduced up to less than 10%, less than 9%, less than 8%, less than 7%, less than 6%, less than 5%, less than 4%, less than 3%, less than 2% or less than 1%, and preferably less than 3%, less than 2%, or less than 1%.

In contrast, in the method of the invention, the proliferation and/or differentiation of endocrine progenitor cells or cells at a later stage of differentiation are not or less affected; and the absolute number of desired insulin-producing cells or pancreatic beta cells in the cell population obtained after differentiation induction does not significantly differ from that of cells differentiated without being embedded into a gel containing alginic acid. Owing the method of the invention, the number of desired insulin-producing cells or pancreatic beta cells in the cell population obtained after differentiation induction can be increased. More specifically, the proportion of desired insulin-producing cells or pancreatic beta cells in the cell population obtained after differentiation induction can be increased compared to that of the cells differentiated without being embedded into a gel containing alginic acid. The ratio can be increased up to 40% or more, 50% or more, 60% or more, 70% or more, 80% or more, or 90% or more.

If the insulin-producing cells or pancreatic beta cells obtained by the method of the invention are transplanted into a site within a living body of animal and differentiated therein, the cells allowed to remain as they are and can be used as insulin-secreting cells.

The insulin-producing cells or pancreatic beta cells obtained by the method of the invention, or a cell population embedded into a gel containing alginic acid according to the present invention can be transplanted into a site within a living body of animal and used as medicaments such as diabetes (1 type diabetes, 2 type diabetes) drugs and a hypoglycemia inhibitor.

The cell population embedded into a gel containing alginic acid and transplanted can avoid or decrease interference from the immune system of a host and the cells of the population can differentiate, proliferate, and/or be maintained in the host organism.

Now, the present invention will be described by way of Examples; however, the present invention is not limited to these Examples.

### Examples

### Example I: Decrease/proliferation inhibition (I) of undesired cells (Ki67-positive cells) in graft by transplantation of endocrine progenitor cell embedded into a gel containing alginic acid

### 1. Method

### (1) Embedding of endocrine progenitor cells into gel containing alginic acid

Endocrine progenitor cells, which were prepared from human iPS cells by differentiation induction were mixed with a 3 wt% alginic acid (PRONOVA SLG100) solution. The resultant cell dispersion was placed in a round mold having an inner diameter of 1 cm² and a thickness of about 500 µm. Cross-links were formed by use of strontium to prepare a gel sheet containing alginic acid into which cells were dispersedly embedded.

### (2) Transplantation of endocrine progenitor cells into living organism

Immunodeficient NOD/SCID mice were used. A transplantation site thereof was treated with bFGF in accordance with a known method to induce angiogenesis and thereafter, the endocrine progenitor cell-embedded gel sheet obtained in step (1) above was subcutaneously transplanted into the site of the back. As Comparative Example, pancreatic endocrine progenitor cells not embedded into a gel containing alginic acid were transplanted into a site under the renal capsule. On day 69 and 6 months after transplantation, grafts were separately excised out.

### (3) Evaluation of undesired cells in graft

The grafts excised out on day 69 after transplantation were fixed, dewatered, and then, frozen sections were prepared. The sections were subjected to immunostaining in order to evaluate undesired cells. Desired cells, which were expected to mature into insulin-secreting cells, were evaluated based on being Chromogranin A (CHGA) positive/NKX6.1 positive; whereas undesired cells, which have a possibility of adversely affecting long-term engraftment of desired cells, were evaluated based on being PDX1 weak-positive or negative/Ki67 positive, as an indicator.

The grafts excised out at 6 months after transplantation were treated with a 100 mM aqueous sodium citrate solution. In this manner, cross-links of alginic acid were deformed and cells were recovered. The cells recovered were treated with 0.25% trypsin-EDTA, dispersed, fixed and subjected to flow cytometry. The undesired cells were evaluated based on being Ki67 positive/insulin negative as an indicator; whereas desired cells were evaluated based on being insulin positive/NKX6.1 positive and glucagon positive/insulin negative as an indicator. Note that, in the specification, the expressions such as "Ki67 positive/insulin negative", "insulin positive/NKX6.1 positive" and "glucagon positive/insulin negative" employ the symbol "/", which means "and".

### 2. Results

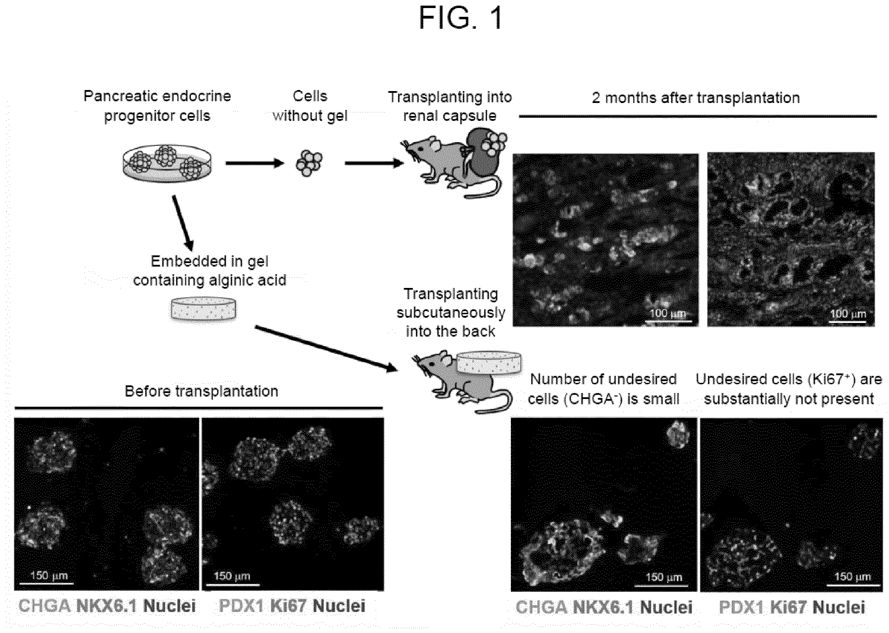

The immunostaining results of the grafts excided out on day 69 after transplantation are shown in Figure 1. It was confirmed that in the case where endocrine progenitor cells were embedded into a gel containing alginic acid and subcutaneously transplanted, the number of Ki67-positive cells contained in the graft is about 1/10 of the number of the cells transplanted into a site under the renal capsule without being embedded into a gel containing alginic acid. From the result, it was demonstrated that the number of Ki67-positive cells decreases or the proliferation thereof can be inhibited by transplanting the endocrine progenitor cells embedded into a gel containing alginic acid.

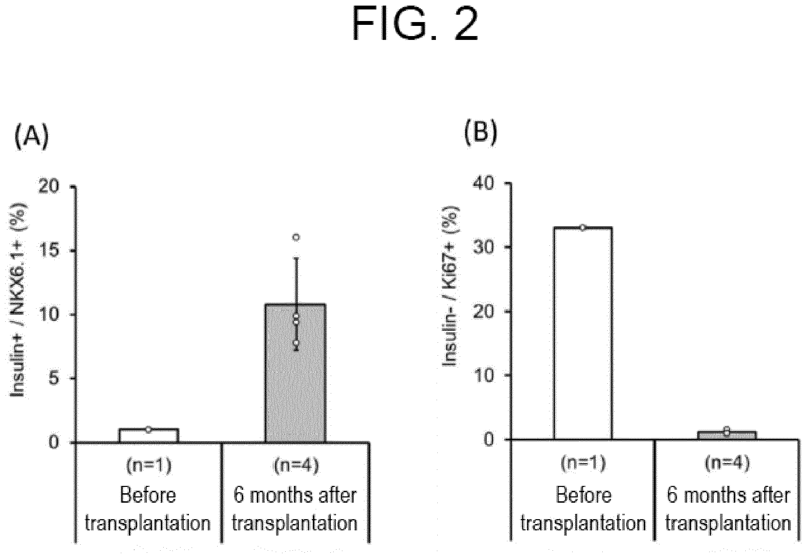

The results of flow cytometric analysis of the cells recovered from the grafts excised out 6 months after transplantation are shown in Figure 2. It was confirmed that endocrine progenitor cells, which were embedded into a gel containing alginic acid and subcutaneously transplanted, are differentiated into desired cells, i.e., insulin positive/NKX6.1 positive cells (Figure 2 (A), 1.0% (n = 1) before transplantation, 10.8 ± 3.6% (n = 4) after transplantation); at the same time, the number of undesired cells, i.e., Ki67 positive/insulin negative cells, dramatically decreases (Figure 2 (B), 33.0% (n = 1) before transplantation, 1.2 ± 0.3% (n = 4) after transplantation).

### Example II: Decrease of undesired proliferating cells after insulin-producing cells including proliferating cells embedded into a gel containing alginic acid were transplanted.

### 1. Method

### (1) Embedding of insulin-producing cells into gel containing alginic acid

Insulin-producing cells, which were prepared from human iPS cells by differentiation induction, were mixed with a 3 wt% alginic acid (PRONOVA SLG100) solution. The resultant cell dispersion was placed in a round mold having an inner diameter of 1 cm² and a thickness of about 500 µm. Cross-links were formed by use of strontium to prepare a gel sheet containing alginic acid into which cells were dispersedly embedded.

### (2) Transplantation of insulin-producing cells into living organism

Immunodeficient NOD/SCID mice were used. The gel sheet having insulin-producing cells embedded thereinto (obtained in step (1) above) was subcutaneously transplanted into a site of the back. Six months after transplantation, grafts were separately excised out.

### (3) Evaluation of undesired cells in graft

The grafts excised out at 6 months after transplantation were treated with a 100 mM aqueous sodium citrate solution. In this manner, cross-links of alginic acid were deformed and cells were recovered. The cells recovered were treated with 0.25% trypsin-EDTA, dispersed, fixed and subjected to flow cytometry. The undesired cells were evaluated based on being Ki67 positive/insulin negative as an indicator; whereas desired cells were evaluated based on being insulin positive/NKX6.1 positive and glucagon positive/insulin negative as an indicator.

### 2. Results

The grafts excised out 6 months after transplantation maintained the same appearance as that before transplantation. The results of flow cytometric analysis of the cells recovered from the grafts excised out are shown in Table 1. It was confirmed that in the case where insulin-producing cells including proliferating cells were embedded into a gel containing alginic acid and subcutaneously transplanted, the number of undesired proliferating cells present before transplantation remarkably decreases. It was simultaneously confirmed that the proportion of glucagon positive/insulin negative cells increased. From this, differentiation into islet-like cells was confirmed.

**[Table 1]**

| | Before transplantation | 6 months after transplantation (N=2) |
|---|---|---|
| Proportion of Ki67 positive/insulin negative cell | 18.6% | 1.8%, 1.3% |
| Proportion of insulin positive/NKX6.1 positive cell | 27.8% | 6.7%, 5.0% |
| Proportion of glucagon positive/insulin negative cell | 0% | 59.2%, 48.6% |

Example III: Decrease (I) of undesired cells (Ki67-positive cells) by *in vitro* culture of insulin-producing cells embedded into a gel containing alginic acid 1. Method

### (1) Embedding of insulin-producing cells into gel containing alginic acid

Insulin-producing cells, which were prepared from human iPS cells by differentiation induction, were mixed separately with alginic acid solutions different in concentration (0.5-3 wt%) and grade (PRONO VA SLG100 (G/M ratio: ≥ 1.5), NOVATACH MVG GRGDSP (G/M ratio: ≥ 1.5) and PRONOVA UP VLVM (G/M ratio: ≤ 1)), in accordance with the formulations specified in Table 2. The resultant cell dispersions each were placed in a round mold having an inner diameter of 1 cm² and a thickness of about 500 µm. Cross-links were formed by use of an aqueous strontium solution or an aqueous calcium solution to prepare a gel sheet containing alginic acid into which cells were dispersedly embedded. The elasticity (Pa) was measured by a rheometer (viscoelasticity measuring device).

**[Table 2]**

| | Alginic acid Grade | Concentration (wt%) | Cross-linking agent | Thickness (*µ*m) | Elasticity (Pa) |
|---|---|---|---|---|---|
| Example 1 | PRONOVA | 0.5 | 70 mM | 129 | 5425 |
| | SLG100 | | Aqueous strontium chloride solution | | |
| Example 2 | PRONOVA | 1.5 | 70 mM | 384 | 5419 |
| | SLG100 | | Aqueous strontium chloride solution | | |
| Example 3 | PRONOVA | 3 | 70 mM | 576 | 8772 |
| | SLG100 | | Aqueous strontium chloride solution | | |
| Example 4 | NOVATACH | 3 | 70 mM | 506 | 7942 |
| | MVG GRGDSP | | Aqueous strontium chloride solution | | |
| Example 5 | PRONOVA UP | 3 | 70 mM | 633 | 2505 |
| | VLVM | | Aqueous strontium chloride solution | | |
| Example 6 | PRONOVA | 3 | 70 mM | 764 | 2777 |
| | SLG100 | | Aqueous calcium chloride solution | | |

### (2) In vitro culture of insulin-producing cells

Insulin-producing cells embedded into hydrogel and insulin-producing cells not embedded into hydrogel (Comparative Example) were cultured in a medium containing a growth factor for differentiating the insulin-producing cells and recovered from the gel day 7 after initiation of culture.

### (3) Evaluation of undesired cells

The endocrine progenitor cells embedded into a gel containing alginic acid were treated with a 100 mM aqueous sodium citrate solution. In this manner, cross-links of alginic acid were deformed and cells were recovered. The endocrine progenitor cells recovered were treated with 0.25% trypsin-EDTA, dispersed and fixed.-The number of Ki67-positive cells was determined by flow cytometry and the rate of change in the number before and after culture was compared.

### 2. Results

Table 3 shows the change rates in the number of Ki67-positive cells before and after culture.

The change rate of cell number in Comparative Example increased by about + 20%; whereas, almost no change was observed in Examples.

**[Table 3]**

| | Change rate in the number of Ki67 positive cells |
|---|---|
| Comparative Example | +22.2% |
| Example 1 | -0.6% |
| Example 2 | -1.4% |
| Example 3 | +1.8% |
| Example 4 | -1.8% |
| Example 5 | -2.1% |
| Example 6 | +1.3% |

### Example IV: Decrease (II) of undesired cells (Ki67-positive cells) by in vitro culture of insulin-producing cells embedded into a gel containing alginic acid 1. Method

### (1) Embedding of insulin-producing cells into gel containing alginic acid

Insulin-producing cells, which were prepared from human iPS cells by differentiation induction, were mixed with the alginic acid solution, PRONOVA SLG100 (G/M ratio: ≥ 1.5), so as to satisfy the cell densities specified in Table 4. Each of the resultant cell dispersions was placed in a round mold having an inner diameter of 1 cm² and a thickness of about 500 µm. Cross-links were formed by use of strontium to prepare a gel sheet containing alginic acid into which cells were dispersedly embedded.

### (2) in vitro culture of insulin-producing cells

Insulin-producing cells embedded into hydrogel and insulin-producing cells not embedded into hydrogel (Comparative Example) were cultured in a medium containing a growth factor for differentiating the insulin-producing cells and in the oxygen conditions specified in Table 4 and recovered day 6 after initiation of culture.

**[Table 4]**

| Number of cell populations/gel sheet | | 1,000 | 3,000 | 6,000 | 9,000 | 12,000 |
|---|---|---|---|---|---|---|
| Oxygen condition in culture chamber | 21% | Example 7 | Example 8 | Example 9 | Example 10 | Example 11 |
| | 5% | Example 12 | Example 13 | Example 14 | Example 15 | Example 16 |
| | 1% | Example 17 | Example 18 | - | - | - |

### (3) Evaluation of undesired cells

The endocrine progenitor cells embedded into a gel containing alginic acid were treated with a 100 mM aqueous sodium citrate solution. In this manner, cross-links of alginic acid were deformed and cells were recovered from the gel. The endocrine progenitor cells were treated with 0.25% trypsin-EDTA, dispersed and fixed. The number of Ki67-positive cells was determined by flow cytometry and the rate of change in the number before and after culture was compared.

### 2. Results

Table 5 shows the change rates in the number of Ki67-positive cells before and after culture.

The change rate of cell number in Comparative Example increased by about 5%; whereas, the change rates of Examples all decreased.

**[Table 5]**

| | Change rate in the number of Ki67 positive cells |
|---|---|
| Comparative Example | +4.8% |
| Example 7 | -1.1% |
| Example 8 | -6.3% |
| Example 9 | -8.3% |
| Example 10 | -7.8% |
| Example 11 | -6.5% |
| Example 12 | -4.9% |
| Example 13 | -6.0% |
| Example 14 | -9.5% |
| Example 15 | -6.9% |
| Example 16 | -7.4% |
| Example 17 | -6.3% |
| Example 18 | -6.1% |

### Example V: Decrease of undesired cells (Ki67-positive cells) by in vitro culture of insulin-producing cells embedded into a gel containing alginic acid and extracellular matrix

### 1. Method

### (1) Embedding of insulin-producing cells into gel containing alginic acid and extracellular matrix

Insulin-producing cells, which were prepared from human iPS cells by differentiation induction, were mixed separately with alginic acid solutions different in grade (PRONOVA SLG100 (G/M ratio: ≥ 1.5), NOVATACH MVG GRGDSP (G/M ratio: ≥ 1.5), NOVATACH M REDV (G/M ratio: ≤ 1), and NOVATACH G VAPG (G/M ratio: ≥ 1.5)), and Hystem, Hystem-C, Hystem-HP or Gelatin in accordance with the formulations specified in Table 6. The resultant cell dispersions each were placed in a round mold having an inner diameter of 1 cm² and a thickness of about 500 µm. Cross-links were formed by use of an aqueous strontium solution and/or an aqueous polyethylene glycol diacrylate solution to prepare gel sheets containing alginic acid and extracellular matrix into which cells were dispersedly embedded.

**[Table 6]**

| | Alginic acid grade | Component | Mixing ratio (v/v) | Cross-linking agent |
|---|---|---|---|---|
| Example 19 | PRONOVA SLG100 (concentration (wt%): 3%) | - | - | 70 mM aqueous strontium chloride solution |
| Example 20 | | Hystem | 1/1 | 70 mM aqueous strontium chloride solution 250 µg/mL aqueous polyethylene glycol diacrylate solution |
| Example 21 | | Hystem-C | 1/1 | |
| Example 22 | | Hystem-HP | 1/1 | |
| Example 23 | | Gelatin | 1/1 | 70 mM aqueous strontium chloride solution |
| Example 24 | NOVATACH MVG GRGDSP (concentration (wt%): 3%) | - | - | 70 mM aqueous strontium chloride solution |
| Example 25 | NOVATACH M REDV (concentration (wt%): 3%) | - | - | 70 mM aqueous strontium chloride solution |
| Example 26 | NOVATACH G VAPG (concentration (wt%): 3%) | - | - | 70 mM aqueous strontium chloride solution |

### (2) In vitro culture of insulin-producing cells

Insulin-producing cells embedded into hydrogel and insulin-producing cells not embedded into hydrogel (Comparative Example) were cultured in a medium containing a growth factor for differentiating the insulin-producing cells and recovered day 7 after initiation of culture.

### (3) Evaluation of undesired cells

The insulin-producing cells embedded into a gel containing alginic acid and extracellular matrix were treated with a 100 mM aqueous sodium citrate solution. In this manner, cross-links of alginic acid were deformed and cells were recovered from the gel. The insulin-producing cells recovered were treated with 0.25% trypsin-EDTA, dispersed and fixed. The number of Ki67-positive cells was determined by flow cytometry and the rate of change in the number before and after culture was compared.

### 2. Results

Table 7 shows the change rates in the number of Ki67-positive cells before and after culture.

The change rate of cell number in Comparative Example increased by about + 5.2%; whereas, the change rates in Examples were almost zero or decreased. In the case where extracellular matrix, which can serve as a scaffold of cells was added to the gel, it was confirmed that the proliferation of Ki67-positive cells can be inhibited.

**[Table 7]**

| | Change rate of Ki67 positive cell number |
|---|---|
| Comparative Example | +5.2% |
| Example 19 | -2.5% |
| Example 20 | -1.3% |
| Example 21 | -1.5% |
| Example 22 | -0.5% |
| Example 23 | -0.1% |
| Example 24 | -3.2% |
| Example 25 | +0.2% |
| Example 26 | -2.0% |

### Example VI: Evaluation of iPS cell proliferation in alginic acid capsule containing nanofiber

Human iPS cells were mixed with a solution in which 1 wt% nanofiber ("BiNFi-s)" (Sugino); grade AFo-100, average fiber diameter 10-50 nm) and 3 wt% alginic acid (PRONOVA SLG100) were added. The obtained mixed solution was added dropwise in a barium chloride solution to prepare nanofiber-containing alginic acid gel capsules having iPS cells embedded thereinto.

The resultant gel capsules were added to a culture medium and the proliferation potency of iPS cells embedded thereinto was examined. As a result, it was confirmed that stable cell mass is formed while suppressing cell leakage by proliferation compared to alginic acid capsules not containing a nanofiber.

## Claims

1. A method for producing an insulin-producing cell population or a pancreatic beta cell population containing less than 3% of Ki67-positive cells, comprising the steps of:
(1) embedding an insulin-producing cell population into a gel containing alginic acid; and (2) differentiating the cell population embedded.

2. The method according to claim 1, wherein the gel in step (1) further contains a nanofiber.

3. The method according to claim 1 or 2, wherein the differentiation in step (2) is performed by transplantation to an animal.

4. A method for decreasing the number of Ki67-positive cells present in an endocrine progenitor cell population or a cell population at a later stage of differentiation or a method for inhibiting the proliferation of the positive cells, comprising
embedding the cell population into a gel containing alginic acid.

5. The method according to claim 4, wherein the number of the endocrine progenitor cells or the cells at a later stage of differentiation, present in the cell population is not decreased.

6. The method according to claim 4 or 5, wherein the gel further contains a nanofiber.

7. A gel containing alginic acid into which an insulin-producing cell population or a pancreatic beta cell population containing less than 3% of Ki67-positive cells is embedded.

8. The gel according to claim 7, wherein the Ki67-positive cells of the cell population are less than 1%.

9. The gel according to claim 7, wherein the cell population is a cell population derived from pluripotent stem cells.

10. The gel according to claim 7, for use in a method for treating diabetes.

11. A method for treating diabetes by immobilizing a gel containing alginic acid into which an insulin-producing cell population or a pancreatic beta cell population containing less than 3% of Ki67-positive cells is embedded, within a living body.

12. A gel containing alginic acid for inhibiting the proliferation of Ki67-positive cells present in an endocrine progenitor cell population or a cell population at a later stage of differentiation.

13. Use of gel containing alginic acid in producing a medicament containing an insulin-producing cell population or a pancreatic beta cell population containing less than 3% of Ki67-positive cells.

14. The method according to claim 1, which is a method for producing an insulin-producing cell population or a pancreatic beta cell population containing less than 1% of Ki67-positive cells.

15. A method for producing an insulin-producing cell population or a pancreatic beta cell population containing less than 3% of Ki67-positive cells, comprising the steps of:
(1) purifying cells;
(2) embedding an insulin-producing cell population into a gel containing alginic acid; and
(3) differentiating the cell population embedded.
